(19) Europäisches Patentamt / European Patent Office / Office européen des brevets

(11) **EP 3 519 820 B1**

(12) **EUROPEAN PATENT SPECIFICATION**

(45) Date of publication and mention of the grant of the patent:
**09.12.2020 Bulletin 2020/50**

(51) Int Cl.:
***G01N 33/543*** *(2006.01)*    ***G01N 33/68*** *(2006.01)*

(21) Application number: **17771434.2**

(22) Date of filing: **21.09.2017**

(86) International application number:
**PCT/EP2017/073848**

(87) International publication number:
**WO 2018/060035 (05.04.2018 Gazette 2018/14)**

(54) **SPR-BASED DUAL-BINDING ASSAY FOR THE FUNCTIONAL ANALYSIS OF MULTISPECIFIC MOLECULES**

SPR-BASIERTER DOPPELBINDUNGSASSAY ZUR FUNKTIONALEN ANALYSE MULTISPEZIFISCHER MOLEKÜLE

ESSAI DE LIAISON DOUBLE À BASE DE SPR POUR L'ANALYSE FONCTIONNELLE DE MOLÉCULES MULTISPÉCIFIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2016 EP 16191712**

(43) Date of publication of application:
**07.08.2019 Bulletin 2019/32**

(73) Proprietor: **F. Hoffmann-La Roche AG**
**4070 Basel (CH)**

(72) Inventors:
• **GASSNER, Christian**
**82377 Penzberg (DE)**
• **MESCHENDOERFER, Wolfgang**
**82377 Penzberg (DE)**
• **MOELLEKEN, Joerg**
**80686 München (DE)**

(74) Representative: **Skolaut, Alexander**
**Roche Diagnostics GmbH**
**Patentabteilung TR-E**
**Nonnenwald 2**
**82377 Penzberg (DE)**

(56) References cited:
**WO-A1-2015/172800    WO-A2-2012/023053**

• **MESCHENDOERFER W ET AL: "SPR-based assays enable the full functional analysis of bispecific molecules", JOURNAL OF PHARMACEUTICAL AND BIOMEDICAL ANALYSIS, vol. 132, 26 September 2016 (2016-09-26), pages 141-147, XP029801513, ISSN: 0731-7085, DOI: 10.1016/J.JPBA.2016.09.028**

**Description**

The current invention is in the field of functional assays. Herein is reported a novel SPR-based dual-binding assay for measuring the interactions of multispecific antibodies. The overall binding can be calculated based on the determined individual readouts.

**Background of the Invention**

SPR (surface plasmon resonance) is a biosensor-based technology to measure real time protein-protein interaction. SPR technology has become a standard tool in biopharmaceutical research and development [1-5], and is commonly employed to determine binding constants for macromolecular interactions. The ability to determine association and dissociation kinetics for molecular interactions provides detailed insights into the mechanism of complex formation [6]. This information is becoming an essential part of the selection and optimization process for monoclonal antibodies and other biopharmaceutical products [7-10]. In addition, SPR technology allows the determination of the binding activity (binding capacity) of e.g. an antibody binding a target.
Only a couple of technologies are available which allow the functional assessment of two or more interactions in one approach (e.g. suspension array technology, reviewed in [12]; time-resolved fluorescence assay [13]). These technologies take advantage of the parallel detection of different fluorophores. In addition, optical biosensors exist, which allow the online - and therefore sequential - measurement of multiple interactions [2-5].
US 2010/256338 disclosed multispecific, especially bispecific antibodies comprising full length antibodies and single chain Fab fragments, methods for their production, pharmaceutical compositions containing the antibodies, and uses thereof.
WO 2012/023053 disclosed novel bispecific monoclonal antibodies carrying a different specificity for each binding site of the immunoglobulin molecule and methods for producing novel bispecific monoclonal antibodies carrying a different specificity for each binding site of the immunoglobulin molecule.
WO 2015/172800 disclosed novel multispecific molecules and novel treatment methods based on such multispecific molecules.
Meschendoerfer, W. et al., disclosed SPR-based assays enable the full functional analysis of bispecific molecules (J. Pharm. Biomed. Anal. 132 (2016) 141-147).

**Summary of the Invention**

Herein is reported a novel SPR-based dual-binding assay for measuring both binding activities of bispecific antibodies. The overall binding can be calculated based on both measured individual readouts. A comparison with a standard SPR-based bridging assay showed good correlation. The assay as reported herein allows a full functional analysis of bispecific antibodies.
One aspect as reported herein is an assay or method for determining the individual and overall binding of a (monoclonal) antibody, which comprises a first binding site specifically binding to a first antigen and a second binding site specifically binding to a second antigen, to said first and said second antigen, wherein the method comprises the following steps:

a) capturing the antibody on a solid phase using a capture reagent specifically binding to a constant domain of the antibody,

b) incubating the captured antibody with the first or the second antigen in a buffer comprising 1 mM KH2PO4, 10 mM Na2HPO4, 137 mM NaCl, 2.7 mM KCl, pH 7.4, 0.05% Tween-20 and 300 mM NaCl to form a captured antibody-antigen complex and determining a first binding signal,

c) either
incubating the captured antibody-antigen complex with the antigen not used for the formation of the captured antibody-antigen complex to form a captured antibody-antigen-antigen complex and determining a second binding signal,
or
regenerating the surface, capturing the antibody on a solid phase using a capture reagent specifically binding to a constant domain of the antibody, incubating the captured antibody with the antigen not used for the formation of the captured antibody-antigen complex in step b) in the same buffer as in step b) to form a second captured antibody-antigen complex and determining a third binding signal,
and

d) determining the overall or individual binding of the antibody to the first and the second antigen if i) the formation of the captured antibody-antigen complex results in a first binding signal and ii) the formation of the captured antibody-antigen-antigen complex or the formation of the (second) captured antibody-antigen complex after regeneration results in a second or third binding signal that is different or increased with respect to the first binding signal, wherein the solid phase is a surface plasmon resonance chip, the first binding signal is a surface plasmon resonance response and the second binding signal is a surface plasmon resonance response.

The binding signal is determined by surface plasmon resonance.

In one embodiment the first antigen or/and the second antigen is a/are soluble antigen(s).

In one embodiment the antibody is a bispecific antibody. In one embodiment the bispecific antibody is a Cross-Mab or a DutaFab.

In one embodiment the capture reagent is an anti-Fc-region antibody or an anti-Fab antibody. In one embodiment the anti-Fab antibody is an anti-kappa-light-chain antibody or an anti-lambda-light-chain antibody.

In one embodiment the antibody is a DutaFab, the solid phase is a surface plasmon resonance chip, the capture reagent is an anti-kappa-light chain antibody or an anti-lambda light chain antibody, the first binding signal is a surface plasmon resonance response and the second binding signal is a surface plasmon resonance response.

In one embodiment the capturing is by injecting the antibody for 45 to 720 seconds, at a flow rate of 2.5 to 10 $\mu$L/min, and at a concentration of 0.6 $\mu$g/mL to 10 $\mu$g/mL. In one embodiment the capturing is by injecting the antibody for about 60 seconds, at a flow rate of about 5 $\mu$L/min, and at a concentration of 0.6 $\mu$g/mL to 10 $\mu$g/mL.

In one embodiment the incubating is by an injection of the respective antigen at a concentration of 0.5 to 5 $\mu$g/mL for 20 to 90 seconds. In one embodiment the incubating is by an injection of the respective antigen at a concentration of about 2 $\mu$g/mL for 30 or 60 seconds.

In one embodiment the antibody is a DutaFab, the solid phase is a surface plasmon resonance chip, the capture reagent is an anti-kappa-light chain antibody or an anti-lambda light chain antibody, the first binding signal is a surface plasmon resonance response, the second binding signal is a surface plasmon resonance response, the immobilizing is by injecting the antibody for about 60 seconds, at a flow rate of about 5 $\mu$L/min, and at a concentration of 0.6 $\mu$g/mL to 10 $\mu$g/mL, and the incubating is by an injection of the respective antigen at a concentration of about 2 $\mu$g/mL for 30 or 60 seconds.

## Detailed Description of the Invention

### Definitions

The term "about" denotes a range of +/- 20 % of the thereafter following numerical value. In one embodiment the term about denotes a range of +/- 10 % of the thereafter following numerical value. In one embodiment the term about denotes a range of +/-5 % of the thereafter following numerical value.

The term "antibody" herein is used in the broadest sense and encompasses various antibody structures, including but not limited to monoclonal antibodies, multispecific antibodies (e.g. bispecific antibodies, trispecific antibodies), and antibody fragments so long as they exhibit the desired antigen-binding activity.

An antibody in general comprises two so called light chain polypeptides (light chain) and two so called heavy chain polypeptides (heavy chain). Each of the heavy and light chain polypeptides contains a variable domain (variable region) (generally the amino terminal portion of the polypeptide chain) comprising binding regions that are able to interact with an antigen. Each of the heavy and light chain polypeptides comprises a constant region (generally the carboxyl terminal portion). The constant region of the heavy chain mediates the binding of the antibody i) to cells bearing a Fc gamma receptor (FcγR), such as phagocytic cells, or ii) to cells bearing the neonatal Fc receptor (FcRn) also known as Brambell receptor. It also mediates the binding to some factors including factors of the classical complement system such as component (C1q). The constant domains of an antibody heavy chain comprise the CHI-domain, the CH2-domain and the CH3-domain, whereas the light chain comprises only one constant domain, CL, which can be of the kappa isotype or the lambda isotype.

The variable domain of an immunoglobulin's light or heavy chain in turn comprises different segments, i.e. four framework regions (FR) and three hypervariable regions (HVR).

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG$_1$, IgG$_2$, IgG$_3$, IgG$_4$, IgA$_1$, and IgA$_2$. The heavy chain constant domains that correspond to the different classes of immunoglobulins are called $\alpha$, $\delta$, $\varepsilon$, $\gamma$, and $\mu$, respectively.

The term "binding (to an antigen)" denotes the binding of an antibody to its antigen in an in vitro assay, in one embodiment in a binding assay in which the antibody is bound to a surface and binding of the antigen to the antibody is measured by Surface Plasmon Resonance (SPR). Binding means the measurement of the binding capacity of e.g.

the antibody for target A or target B, or for a capture molecule e.g. anti-human-Fab-capture for the antibody.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));

(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));

(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and

(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to Kabat et al.

The term "valent" as used within the current application denotes the presence of a specified number of binding sites in a (antibody) molecule. As such, the terms "bivalent", "tetravalent", and "hexavalent" denote the presence of two binding site, four binding sites, and six binding sites, respectively, in a (antibody) molecule. The bispecific antibodies as reported herein are in one preferred embodiment "bivalent".

Multispecific antibodies

In certain embodiments, the antibody is a multispecific antibody, e.g. a bispecific antibody. Multispecific antibodies are monoclonal antibodies that have binding specificities for at least two different sites. In certain embodiments, one of the binding specificities is for a first antigen and the other is for a different second antigen. In certain embodiments, multispecific antibodies may bind to two different epitopes of the same antigen. Multispecific antibodies may also be used to localize cytotoxic agents to cells, which express the antigen. Multispecific antibodies can be prepared as full-length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A., et al., EMBO J. 10 (1991) 3655-3659), and "knob-in-hole" engineering (see, e.g., US 5,731,168). Multi-specific antibodies may also be made by engineering electrostatic steering effects for making antibody Fc-heterodimeric molecules (WO 2009/089004); cross-linking two or more antibodies or fragments (see, e.g., US 4,676,980, and Brennan, M., et al., Science 229 (1985) 81-83); using leucine zippers to produce bi-specific antibodies (see, e.g., Kostelny, S.A., et al., J. Immunol. 148 (1992) 1547-1553; using "diabody" technology for making bispecific antibody fragments (see, e.g., Holliger, P., et al., Proc. Natl. Acad. Sci. USA 90 (1993) 6444-6448); and using single-chain Fv (scFv) dimers (see, e.g., Gruber, M., et al., J. Immunol. 152 (1994) 5368-5374); and preparing trispecific antibodies as described, e.g., in Tutt, A., et al., J. Immunol. 147 (1991) 60-69).

The antibody or fragment can also be a multispecific antibody as described in WO 2009/080251, WO 2009/080252, WO 2009/080253, WO 2009/080254, WO 2010/112193, WO 2010/115589, WO 2010/136172, WO 2010/145792, or WO 2010/145793.

The antibody or fragment thereof may also be a multispecific antibody as disclosed in WO 2012/163520 (also referred to as "DutaFab").

Bispecific antibodies are generally antibody molecules that specifically bind to two different, non-overlapping epitopes on the same antigen or to two epitopes on different antigens.

Different bispecific antibody formats are known.

Exemplary bispecific antibody formats for which the methods as reported herein can be used are

- the CrossMab format (=CrossMab): a multispecific IgG antibody comprising a first Fab fragment and a second Fab fragment, wherein in the first Fab fragment

    a) only the CH1 and CL domains are replaced by each other (i.e. the light chain of the first Fab fragment comprises a VL and a CH1 domain and the heavy chain of the first Fab fragment comprises a VH and a CL domain);

    b) only the VH and VL domains are replaced by each other (i.e. the light chain of the first Fab fragment comprises a VH and a CL domain and the heavy chain of the first Fab fragment comprises a VL and a CH1 domain); or

    c) the CH1 and CL domains are replaced by each other and the VH and VL domains are replaced by each other (i.e. the light chain of the first Fab fragment comprises a VH and a CH1 domain and the heavy chain of the first Fab fragment comprises a VL and a CL domain); and
    wherein the second Fab fragment comprises a light chain comprising a VL and a CL domain, and a heavy chain comprising a VH and a CH1 domain;
    the CrossMab may comprises a first heavy chain including a CH3 domain and a second heavy chain including a CH3 domain, wherein both CH3 domains are engineered in a complementary manner by respective amino acid substitutions, in order to support heterodimerization of the first heavy chain and the modified second heavy chain, e.g. as disclosed in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, or WO 2013/096291;

- the one-armed single chain format (= one-armed single chain antibody): antibody comprising a first binding site that specifically binds to a first epitope or antigen and a second binding site that specifically binds to a second epitope or antigen, whereby the individual chains are as follows

    - light chain (variable light chain domain + light chain kappa constant domain)
    - combined light/heavy chain (variable light chain domain + light chain constant domain + peptidic linker + variable heavy chain domain + CH1 + Hinge + CH2 + CH3 with knob mutation)
    - heavy chain (variable heavy chain domain + CH1 + Hinge + CH2 + CH3 with hole mutation);

- the two-armed single chain format (= two-armed single chain antibody): antibody comprising a first binding site that specifically binds to a first epitope or antigen and a second binding site that specifically binds to a second epitope or antigen, whereby the individual chains are as follows

    - combined light/heavy chain 1 (variable light chain domain + light chain constant domain + peptidic linker + variable heavy chain domain + CH1 + Hinge + CH2 + CH3 with hole mutation)
    - combined light/heavy chain 2 (variable light chain domain + light chain constant domain + peptidic linker + variable heavy chain domain + CH1 + Hinge + CH2 + CH3 with knob mutation);

- the common light chain bispecific format (= common light chain bispecific antibody): antibody comprising a first binding site that specifically binds to a first epitope or antigen and a second binding site that specifically binds to a second epitope or antigen, whereby the individual chains are as follows

    - light chain (variable light chain domain + light chain constant domain)
    - heavy chain 1 (variable heavy chain domain + CH1 + Hinge + CH2 + CH3 with hole mutation)
    - heavy chain 2 (variable heavy chain domain + CH1 + Hinge + CH2 + CH3 with knob mutation);

- the DutaFab: antibody comprising two (non-overlapping) paratopes in a complementary pair of a VH and a VL domain, wherein the first paratope comprises (consists of) amino acid residues from CDR1 and CDR3 of the VL domain and CDR2 of the VH domain, and the second paratope comprises (consists of) residues from CDR1 and CDR3 of the VH domain and CDR2 of the VL domain.

The term "non-overlapping" in this context indicates that an amino acid residue that is comprised within the first paratope of the DutaFab is not comprised in the second paratope, and an amino acid that is comprised within the second paratope of the DutaFab is not comprised in the first paratope.

In one embodiment the bispecific antibody is a CrossMab.

In one embodiment the bispecific antibody is a one-armed single chain antibody.

In one embodiment the bispecific antibody is a two-armed single chain antibody.

In one embodiment the bispecific antibody is a common light chain bispecific antibody.

In one embodiment the bispecific antibody is a DutaFab.

Multivalent, multispecific antibodies specifically bind to different targets, most likely with different affinities and complex stabilities for each target. Only a fully active multivalent, multispecific antibody can bind to all targets and shows the full biological activity in a corresponding assay.

The method as reported herein

The increasing complexity of novel biotherapeutics such as bispecific antibodies or fusion proteins raises new challenges for functional characterization. When compared to standard antibodies, two individual interactions need to be considered for bispecific monoclonal antibodies.

Previously an SPR-based assay setup has been described, which allows to assess the binding activity of a bivalent-bispecific molecule to both targets simultaneously and - in addition to one individual target - in a single setup. However, there might be some pitfalls when applying the bridging assay based on the analyzed molecule e.g. a change of antigen activity upon immobilization.

In more detail, an SPR based assay principle has been reported enabling the assessment of the binding activity of a bispecific antibody to both of its targets in parallel [11]. Development of an SPR-based bridging assay enabled to determine overall drug binding to both targets and - in parallel - assessment of the individual binding contribution of one antigen relative to a reference molecule. In theory, the overall binding signal is multiplicatively influenced by both individual binding events, therefore the 2nd individual binding activity can be calculated when the other two variables are known:

$$\text{antigen-2 binding (\%)} = \text{overall binding (\%)} / \text{antigen-1 binding (\%)} \quad \text{(equation 1)}$$

Herein is reported an SPR-based assay, which allows the individual assessment of both targets in solution. This assay overcomes drawbacks of the currently known assay setups. Comparison of data between the assays showed that overall binding can be calculated based on both measured individual readouts. This is confirmed by a good correlation. Hence, the SPR-based assay principle allows a full functional analysis of bispecific monoclonal antibodies, such as a bispecific CrossMab or DutaFab, in only one assay.

Thus, in one embodiment the methods as reported herein is for the individual assessment of the binding of a bispecific antibody to both targets in solution.

The assay can be qualified and allows for an efficient drug development.

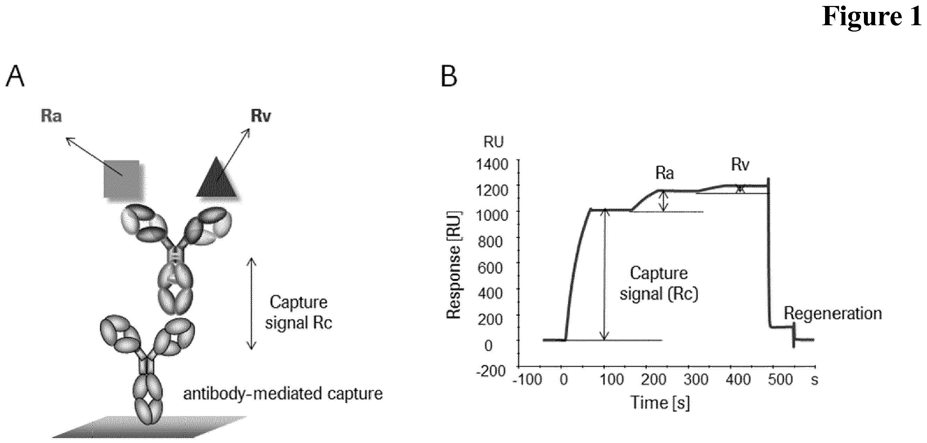

In more detail, herein is reported an SPR-based assay setup, in the following denoted as dual-binding assay. This assay avoids the need of immobilization and regeneration of the target (antigen) by using well-established capturing systems and allows determination of both antibody functionalities directly in a single assay setup. The overall binding can be calculated as follows (see also Figure 1A):

$$\text{overall binding (\%)} = \text{antigen-2 binding (\%)} \times \text{antigen-1 binding (\%)} \quad \text{(equation 2)}$$

Thus, in one embodiment the methods as reported herein are for the determination of both antibody functionalities of a bispecific antibody directly without capture of the antigens to a solid phase.

Thus, in one embodiment the methods as reported herein are for the determination of the overall (i.e. to antigen-1 and antigen-2) binding of a bispecific antibody, wherein the overall binding is calculated according to equation 2.

The current SPR-based assay setup allows obtaining all the measureable binding events of a given bispecific molecule in parallel: two binding events can be measured, and the third parameter can be calculated based on these. This approach will be denoted herein as the "full functional analysis of bispecific molecules".

In addition, the assay as reported herein has been qualified and could show its excellent performance by precision and accuracy determination. This ensures that this assay can be validated and hence be used for later stages of product development.

The herein reported generic assay setup allows determining the individual antigen-1 and antigen-2 binding activities of a bispecific antibody. This is based on an SPR-based method, which allows measurement of both target-binding events to the immobilized bispecific antibody.

Thus, in one embodiment the methods as reported herein is for determining the individual antigen-1 and antigen-2 binding activities of a bispecific antibody.

As capture system an anti-human-Fab capture system was chosen. Advantages of this capture system for assay development is, among other things, the established regeneration conditions, thus avoiding inconsistent measurements over time as bispecific antibody gets captured at each cycle. Because of this advantage, the binding properties of the recruited antibody remain constant during the whole measurement.

Generally there are two principle approaches for obtaining the antigen-2 and the antigen-1 signal in such a setup:

A) First, within one cycle, the antigen-2 binding (Ra) is determined and on top, the antigen-1 binding (Rv) with antigen-2-bound bispecific antibody is determined or vice versa.

B) The antigen-2 binding (Ra) is determined first, and upon regeneration, the second antigen-1 interaction (Rv) is determined with new immobilized bispecific antibody (Rc).

If both interactions occur independently, the two approaches will yield equal results. Approach B) needs an additional capture and regeneration step and increases measurement time significantly. Two different report points can be used as readout: either a report point including the capture level of the bispecific antibody (Rc) and the binding level of the antigen (Ra, Rv), or only the binding signal of the antigen to his antibody (Ra, Rv). In order to avoid the non-biologically relevant contribution of the Fc interaction (Rc), only the binding signal Ra and Rv was used.

Thus, in one embodiment the methods as reported herein use as readout for determining the binding either the capture level of the bispecific antibody (Rc) and the binding level of the antigen (Ra, Rv), or only the individual binding signal of the antigens to the antibody (Ra, Rv).

To demonstrate independent antigen binding to the target, both functionalities were individually measured and compared to those obtained from sequential injections.

Bispecific antibody = CrossMab:

| | Individual binding | | Sequential binding | |
|---|---|---|---|---|
| Sample | antigen-2 binding [%] | antigen-1 binding [%] | antigen-2 Binding [%] | antigen-1 binding [%] |
| Reference | 100 | 100 | 100 | 100 |
| Reference 50% | 57 | 55 | 56 | 54 |
| Reference 150% | 150 | 145 | 147 | 146 |

Bispecific antibody = DutaFab:

| | Sequential binding antigen-1, antigen-2 | | Sequential binding antigen-2, antigen-1 | |
|---|---|---|---|---|
| Sample | antigen-2 binding [%] | antigen-1 binding [%] | antigen-2 Binding [%] | antigen-1 binding [%] |
| Reference | 100 | 100 | 100 | 100 |
| Reference 50% | 50 | 48 | 50 | 48 |
| Reference 150% | 144 | 146 | 147 | 144 |

Thus, the binding signals derived from sequential antigen injection are comparable to those obtained from individual injections. Therefore, both analyzed target-binding events occur independently. The sequential approach is preferred due to shorter measuring time and reduction of regeneration cycles, which may also influence the capacity of the capture system.

Thus, in one embodiment the binding of each antigen to the bispecific antibody is determined to be independent if the binding signals determined in sequential antigen injection are comparable to those obtained from individual injections.

The term "comparable" as used herein denotes in one embodiment a range of +/-20 % of the thereafter following numerical value. In one embodiment the term comparable denotes a range of +/- 10 % of the thereafter following numerical value. In one embodiment the term comparable denotes a range of +/- 5 % of the thereafter following numerical value.

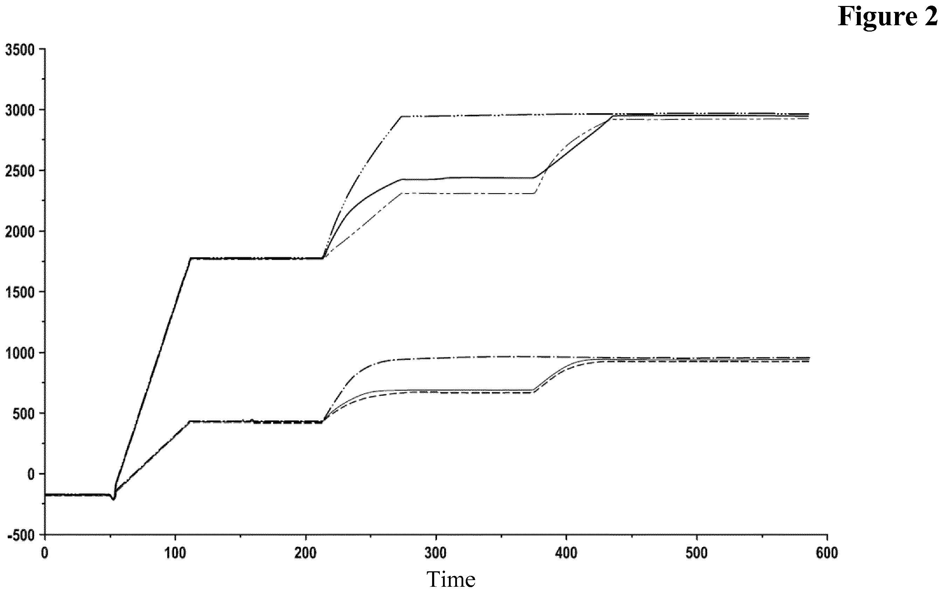

This result is independent of the immobilized bispecific antibody amount (see Figure 2).

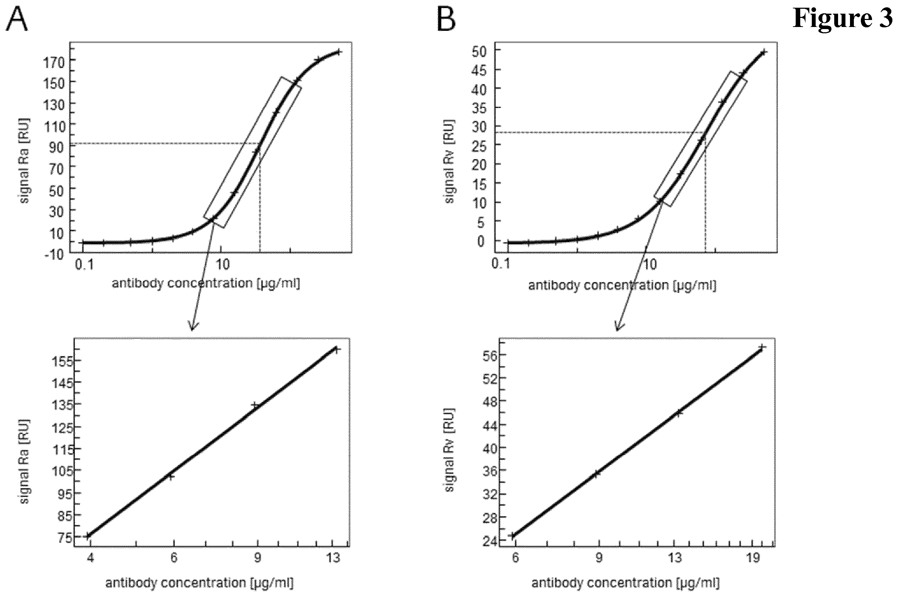

The optimal concentration range for the parallel line analysis can be determined using a dose response curve determined for both antigens. For example, a concentration range of the antibody from 0-1000 $\mu$g/ml can be used for both antigens. A dose-response curve can be generated from sensorgrams with ascending bispecific antibody concentrations and constant antigen-2 and antigen-1 concentrations. The final response Ra for antigen-2 and Rv for antigen-1 of the concentration range each produces a sigmoidal dose response curve. From this dose response curve the optimal linear range around the inflection point can be evaluated (Figure 3).

As the bispecific antibody normally has different affinities for the two antigens, two different linear ranges with a concentration will be obtained. In order to cover the entire range caused by the different linearity ranges of each antigen, a 5 point concentration range can be established starting with the lowest concentration value of the two ranges and ending with the highest concentration value of the two ranges. This allows measurements with both antigens within one cycle. The first four concentrations were used for antigen-2 determination (Ra), whereas the last four concentrations were considered for the antigen-1 determination (Rv). For the qualification of the assay five different ranges from 50% to 150% are used and for each range binding activity are measured in triplicates.

For an exemplary anti-ANG2/VEGF bispecific antibody in CrossMab format two different linear ranges with a concentration of 3.93 - 13.3 $\mu$g/ml for ANG2 (Figure 3A) and 5.9-20 $\mu$g/ml for VEGFA-121 (Figure 3B) were obtained. A 5-point concentration range of 3.93 to 20 $\mu$g/ml was established. The determined relative binding activity calculated from the binding level of each sample divided through the binding level of the standard was plotted against the measured values and linear regression was applied (Figure 3A for Ang2 (Ra) and Figure 3B for VEGFA-121 (Rv)). Results are shown in the Table below.

| Qualification results | Ang2 | VEGFA-121 |
|---|---|---|
| Linearity | 0.9991 | 0.9999 |
| Accuracy | 97% | 99% |
| Precision | 1% | 1% |
| Range | 50-150% | 50-150% |

These data reflect the high performance of the dual-binding assay.

It has been found that in the assay as reported herein the capture of the antibody is best performed at a flow of 5$\mu$l/min for 60 seconds at a concentration of the antibody in the range of 0.6$\mu$g/mL to 10$\mu$g/mL. In this range a linear capture of the antibody can be achieved.

Different buffers have been tested. It has been found that a buffer comprising PBS-T and 300 mM NaCl shows the least non-specific binding at a concentration of 5$\mu$g/ml and below.

| sample | concentration | PBS-T | HBS-P | HBS-EP | PBS-T + 1% BSA | PBS-T + 15 CMD | PBS-T + 300 mM NaCl |
|---|---|---|---|---|---|---|---|
| | 10 | 149 | 171.9 | 122.6 | 141.4 | 171.4 | 6.1 |
| | 5 | 56.1 | 63.8 | 33.2 | 55.4 | 70.6 | 3.6 |
| antigen-2 | 2.5 | 13.3 | 18.1 | 6 | 17 | 24.6 | 2.8 |
| | 1.23 | 6.5 | 8.3 | 2.6 | 7.1 | 9.3 | 2.4 |
| | 0.625 | 3.8 | 6.1 | 2.3 | 3.8 | 4.9 | 2 |

The assay as reported herein is specific and loss-of-function indicating. This was shown using the same bispecific CrossMab as above, an isotype control and two differently stressed CrossMab samples. In order to assess the loss-of-function properties of the assay, CrossMab samples were incubated for moderate or accelerated stress. With the assay as reported herein the loss of binding activity at two different stress conditions could be determined. Additionally, a monoclonal human IgG1 with a different target specificity served as negative control. Thus, the method as reported herein is specific for the drug, loss of function indicating and suitable for later stages of drug development.

| relative binding activity | Ang2 | VEGFA-121 |
|---|---|---|
| reference standard | 100 | 100 |
| moderate stress | 76 | 89 |
| accelerated stress | 59 | 72 |
| isotype control | 4 | 1 |

The assay as reported herein allows that the third not directly measured interaction can be derived from the two analyzed interactions as shown in equation (2) enabling overall signal calculation for the dual-binding assay.

The assays as reported herein shows a good correlation with regard to all possible target interactions (overall, antigen-1, antigen-2), and therefore can be used, e.g. to indicate an independent function loss of the binding sites of a bispecific antibody.

In the previously described bridging assay, complex stability of the antibody bound to the immobilized antigen has to be high enough to permit injection of the second antigen. If antibody dissociation from the surface is occurring too fast, a bridging signal cannot be generated and therefore simultaneous binding of both antigens cannot be achieved. Furthermore, as one of the two antigens has to be immobilized, relatively mild regeneration conditions have to be identified to ensure complete dissociation of the antibody without severe reduction of its activity. Even if a number of regeneration solutions for scouting experiments are available, the protein complex may only be disrupted by use of harsh reagents. This may lead to the damage of the immobilized protein and may significantly reduce surface capacity.

In contrast thereto when using the assay as reported herein (dual binding assay), complex stability of the bispecific molecule with its antigens does not influence the results. Therefore, molecules with low complex stability can be analyzed. An additional advantage of the assay is the capture system applied. Because of this advantage, the binding properties of the surface bound bispecific molecule remain identical during the entire determination while antigen-binding capability remains constant.

The bridging assay and the dual binding assay possess certain features with respect to the analyzed molecules and to the focus of the determination.

A bridging assay is selective for the active binding site related to the immobilized antigen. Molecules, which do not bind to the immobilized antigen are lost, even if the binding site related to the second antigen in solution are fully active. A bridging assay covers both binding events simultaneously and describes the active concentration of all molecules, which shows binding to both of its antigens.

The SPR-based dual binding assay as reported herein requires a capture step, which introduces an additional interaction. Thus, only molecules, which can be captured, are analyzed. The focus of this assay principle is the independent binding of both antigens to the bispecific molecule.

Taken together, the novel SPR-based dual binding assay as reported herein can avoid technical pitfalls caused by a given bispecific antibody. This assay principle measures the interaction of both individual targets, whereas the previously described SPR-based bridging assay principle addresses overall binding to both targets, and in addition, one individual interaction [11].

Reference List

[1] M. A. Cooper, Nature Reviews Drug Discovery 1, (2002) 515-528.

[2] D. G. Myszka, Journal of Molecular Recognition 12, (1999) 390-408.

[3] R. L. Rich, D. G. Myszka, Journal of Molecular Recognition 13, (2000) 388-407.

[4] D. G. Myszka, R. L. Rich, Pharmaceutical science & technology today 3, (2000) 310-317.

[5] R. Karlsson, A. Faelt, Journal of immunological methods 200, (1997) 121-133.

[6] T. A. Morton, D. G. Myszka, Methods in enzymology 295, (1998) 268-294.

[7] K. Nagata, H. Handa, Real-time analysis of biomolecular interactions, Springer, 2000.

[8] R. L. Rich, D. G. Myszka, Current opinion in biotechnology 11, (2000) 54-61.

[9] A. C. Malmborg, C. A. Borrebaeck, Journal of immunological methods 183, (1995) 7-13.

[10] W. Huber, F. Mueller, Current pharmaceutical design 12, (2006) 3999-4021.

[11] C. Gassner, F. Lipsmeier, P. Metzger, H. Beck, A. Schnueriger, J. T. Regula, J. Moelleken, Journal of pharmaceutical and biomedical analysis 102, (2015) 144-149.

[12] Yuankui Leng, Chem. Soc. Rev 44 (2015).

[13] Tian-Cai Liu, Clin. Biochem. 47 (2014) 439-444.

[14] U. S. P. Chapter <1034>, 2012.

The following examples and figures are provided to aid the understanding of the present invention, the true scope of which is set forth in the appended claims.

## Description of the Figures

| Figure 1 | Capturing and interaction of the two antigens to a bispecific antibody, exemplified by a CrossMab. (A) First, the CrossMab is captured by Fab-capture-Antibody (capture signal Rc). Second, antigen-2 (Ra) and/or antigen-1 (Rv) can be recruited from solution to the captured CrossMab. (B) Sensorgram displaying the individual interaction. Rc: Binding response corresponding to capture signal. Ra: Binding response corresponding to antigen-2 binding. Rv: Binding response corresponding to antigen-1 binding. |
| Figure 2 | Independent binding of antigen-2 and antigen-1 determined with a DutaFab captured at 1.5 $\mu$g/mL (lower graphs) and at 5 $\mu$g/mL (upper graph) by injection of antigen-2 and antigen-1 simultaneously or sequentially; the graphs are triplicates whereof the upper one was obtained by the simultaneous injection of antigen-2 and antigen-1, the middle one was obtained by injection of antigen-1 followed by the injection of antigen-2, the lower one was obtained by injection of antigen-2 followed by injection of antigen-1. |
| Figure 3 | Linear dose-response-curve out of a full dose-response curve with different bispecific antibody (CrossMab) concentrations. For a final readout parallel line model was used plotting the antigenbinding signal of (A) antigen-2 against the bispecific antibody concentration on a logarithmic scale and (B) antigen-1 against the bispecific antibody concentration on a logarithmic scale. |

## Examples

## Equipment and reagents

All SPR experiments were performed on a BIAcore©T200 instrument (GE Healthcare) at 25°C. VEGFA-121, Ang2, CrossMabs and all used control antibodies were manufactured by Roche Diagnostics GmbH.

## Example 1

## Assay procedure - Dual binding assay

Capturing anti-human Fab antibody (Human Fab Capture Kit, GE Healthcare) was immobilized on the surface of a CM5 biosensor chip using the provided amine coupling chemistry from GE Healthcare. Flow cells were activated with a 1:1 mixture of 0.4 M 1-ethyl-3-(3-dimethylaminopropyl)-carbodiimide (EDC) and 0.1 M N-hydroxysuccinimide (NHS) at a flow rate of 5 $\mu$L/min. Anti-human Fab antibody was injected in sodium acetate, pH 5.0 at 15 $\mu$g/ml for 420 sec, which resulted in a surface density of approximately 6000 RU. At least 5000 RU drug should be immobilized to ensure target binding is not limited by the immobilization. A reference control flow cell was treated in the same way as described before. Finally both surfaces were blocked with an injection of 1 M ethanolamine/HCl pH 8.5. As immobilization buffer HBS-N (10 mM HEPES, 150 mM NaCl, pH 7.4, GE Healthcare) was used. The bispecific antibodies were diluted in PBS-T (1 mM $KH_2PO_4$, 10 mM $Na_2HPO_4$, 137 mM NaCl, 2.7 mM KCl, pH 7.4, 0.05% Tween-20; Roche Diagnostics GmbH, Mannheim, Germany) and injected on the second flow cell at various concentrations for 90 sec at a flow rate of

10 μL/min. Ang2 was injected for 60 sec with a concentration of 1.1 μg/ml followed by an injection of 1.4 μg/ml VEGFA-121 for 60 sec over both flow cells. The dissociation time (washing with running buffer) was 30 sec at a flow rate of 10 μL/min. All interactions were performed at 25 °C. The regeneration solution of Glycine (pH 2.1) was injected for 60 sec at a flow rate of 30 μL/min to remove any non-covalently bound protein after each binding cycle. Signals were detected at a rate of one signal per second. Samples were measured in triplicates and the activity was measured relative to a standard sample.

### Example2

### Assay procedure - Target-based bridging assay

The target-based bridging assay is essentially based on the assay described in Gassner et al. [11] with the following modifications: The dose-response curve was adjusted to e.g. 1.92-5 μg/ml. The assay could be qualified in the range of 50% to 150%: Linearity was 0.9987, accuracy was 97% and the precision was 2.9%. The assay is specific for the drug, loss of function indicating and suitable for later stage validation.

### Example 3

### Curve fitting and statistical analysis

For the assays a parallel line model according to USP 1034 [14] for relative binding activity calculation was used. The parallel line model can be described by the following equations:

$$Y_S = \alpha_S + \beta \log(x) + e \qquad \text{(equation 3)}$$

$$Y_T = \alpha_T + \beta \log(x) + e \qquad \text{(equation 4)}$$

where $Y_S$ is the response to a standard sample with concentration $x$ and $Y_T$ is the response to a test sample with the same concentration x. The potency of a test sample relative to a standard sample is $= \exp(\frac{\alpha_T - \alpha_S}{\beta})$. The basic similarity assumption for this model is the equality of slopes $\beta$.

### Claims

1. A method for determining the binding of an antibody, which comprises a first binding site specifically binding to a first antigen and a second binding site specifically binding to a second antigen, to said first and said second antigen, wherein the method comprises the following steps:

   a) capturing the antibody on a solid phase using a capture reagent specifically binding to a constant domain of the antibody,
   b) incubating the captured antibody with the first or the second antigen in a buffer comprising 1 mM KH2PO4, 10 mM Na2HPO4, 137 mM NaCl, 2.7 mM KCl, pH 7.4, 0.05% Tween-20 and 300 mM NaCl to form a captured antibody-antigen complex and determining a first binding signal,
   c) either

      incubating the captured antibody-antigen complex with the antigen not used for the formation of the captured antibody-antigen complex to form a captured antibody-antigen-antigen complex and determining a second binding signal,
      or
      regenerating the surface, capturing the antibody on a solid phase using a capture reagent specifically binding to a constant domain of the antibody, incubating the captured antibody with the antigen not used for the formation of the captured antibody-antigen complex in step b) in the same buffer as in step b) to form a captured antibody-antigen complex and determining a third binding signal, and

d) determining the overall or individual binding of the antibody to the first and the second antigen from the first binding signal and the second or third binding signal,

wherein the solid phase is a surface plasmon resonance chip, the first binding signal is a surface plasmon resonance response and the second binding signal is a surface plasmon resonance response.

2. The method according to claim 1, wherein step d) is

d) determining the overall or individual binding of the antibody to the first and the second antigen if i) the formation of the captured antibody-antigen complex results in a first binding signal and ii) the formation of the captured antibody-antigen-antigen complex or the formation of the captured antibody-antigen complex after regeneration results in a second or third binding signal that is increased with respect to the first binding signal.

3. A method for determining overall and individual binding of an antibody, which comprises a first binding site specifically binding to a first antigen and a second binding site specifically binding to a second antigen, to said first and said second antigen, wherein the method comprises the following steps:

- capturing the antibody on a solid phase using a capture reagent specifically binding to a constant region of the antibody,
- incubating the captured antibody with the first or second antigen in a buffer comprising 1 mM KH2PO4, 10 mM Na2HPO4, 137 mM NaCl, 2.7 mM KCl, pH 7.4, 0.05% Tween-20 and 300 mM NaCl to form an immobilized antibody-antigen complex,
- incubating the captured antibody-antigen complex with the antigen not used for the formation of the captured antibody-antigen complex in a buffer comprising 1 mM KH2PO4, 10 mM Na2HPO4, 137 mM NaCl, 2.7 mM KCl, pH 7.4, 0.05% Tween-20 and 300 mM NaCl to form a captured antibody-antigen-antigen complex, and
- determining the individual and based on this, also the overall binding of the antibody to the first and the second antigen if the formation of the captured antibody-antigen complex results in a first binding signal and the formation of the captured antibody-antigen-antigen complex results in a second binding signal that is increased with respect to the first binding signal,

wherein the solid phase is a surface plasmon resonance chip, the first binding signal is a surface plasmon resonance response and the second binding signal is a surface plasmon resonance response.

4. The method according to any one of claims 1 to 3, wherein the antibody is a bispecific antibody.

5. The method according to claim 4, wherein the bispecific antibody is a CrossMab or a DutaFab.

6. The method according to any one of claims 1 to 5, wherein the capture reagent is an anti-Fc-region antibody or an anti-Fab antibody.

7. The method according to claim 6, wherein the anti-Fab antibody is an anti-kappa-light-chain antibody or an anti-lambda-light-chain antibody.

8. The method according to any one of claims 1 to 7, wherein the antibody is a DutaFab, the solid phase is a surface plasmon resonance chip, the capture reagent is an anti-kappa-light chain antibody or an anti-lambda light chain antibody, the first binding signal is a surface plasmon resonance response and the second binding signal is a surface plasmon resonance response.

9. The method according to any one of claims 1 to 8, wherein the capturing is by injecting the antibody for 45 to 720 seconds, at a flow rate of 2.5 to 10 μL/min, and at a concentration of 0.6 μg/mL to 10 μg/mL.

10. The method according to claim 9, wherein the capturing is by injecting the antibody for about 60 seconds, at a flow rate of about 5 μL/min, and at a concentration of 0.6 μg/mL to 10 μg/mL.

11. The method according to any one of claims 1 to 10, wherein the incubating is by an injection of the respective antigen at a concentration of 0.5 to 5 μg/mL for 20 to 90 seconds.

12. The method according to claim 11, wherein the incubating is by an injection of the respective antigen at a concentration of about 2 μg/mL for 30 or 60 seconds.

**Patentansprüche**

1. Verfahren zum Bestimmen der Bindung eines Antikörpers, der eine erste Bindungsstelle, die spezifisch an ein erstes Antigen bindet, und eine zweite Bindungsstelle, die spezifisch an ein zweites Antigen bindet, umfasst, an das erste und das zweite Antigen, wobei das Verfahren die folgenden Schritte umfasst:

   a) Einfangen des Antikörpers auf einer festen Phase unter Verwendung eines Einfangreagenz, das spezifisch an eine konstante Domäne des Antikörpers bindet,
   b) Inkubieren des eingefangenen Antikörpers mit dem ersten oder dem zweiten Antigen in einem Puffer, umfassend 1 mM KH2PO4, 10 mM Na2HPO4, 137 mM NaCl, 2,7 mM KCl, pH 7,4, 0,05 % Tween-20 und 300 mM NaCl, um einen Komplex aus eingefangenem Antikörper und Antigen zu bilden, und Bestimmen eines ersten Bindungssignals,
   c) entweder

      Inkubieren des Komplexes aus eingefangenem Antikörper und Antigen mit dem Antigen, das nicht für die Bildung des Komplexes aus eingefangenem Antikörper und Antigen verwendet wurde, um einen Komplex aus eingefangenem Antikörper, Antigen und Antigen zu bilden, und Bestimmen eines zweiten Bindungssignals,
      oder

      Regenerieren der Oberfläche, Einfangen des Antikörpers auf einer festen Phase unter Verwendung eines Einfangreagenz, das spezifisch an eine konstante Domäne des Antikörpers bindet, Inkubieren des eingefangenen Antikörpers mit dem Antigen, das nicht für die Bildung des Komplexes aus eingefangenem Antikörper und Antigen in Schritt b) verwendet wurde, in demselben Puffer wie in Schritt b), um einen Komplex aus eingefangenem Antikörper und Antigen zu bilden, und Bestimmen eines dritten Bindungssignals, und

   d) Bestimmen der Gesamt- oder Einzelbindung des Antikörpers an das erste und das zweite Antigen aus dem ersten Bindungssignal und dem zweiten oder dritten Bindungssignal,
   wobei die feste Phase ein Oberflächenplasmonenresonanzchip ist, das erste Bindungssignal eine Oberflächenplasmonenresonanzantwort ist und das zweite Bindungssignal eine Oberflächenplasmonenresonanzantwort ist.

2. Verfahren nach Anspruch 1, wobei Schritt d) Folgendes ist
   d) Bestimmen der Gesamt- oder Einzelbindung des Antikörpers an das erste und das zweite Antigen, wenn i) die Bildung des Komplexes aus eingefangenem Antikörper und Antigen zu einem ersten Bindungssignal führt und ii) die Bildung des Komplexes aus eingefangenem Antikörper, Antigen und Antigen oder die Bildung des Komplexes aus eingefangenem Antikörper und Antigen nach Regeneration zu einem zweiten oder dritten Bindungssignal führt, das gegenüber dem ersten Bindungssignal erhöht ist.

3. Verfahren zur Bestimmung der Gesamt- und Einzelbindung eines Antikörpers, der eine erste Bindungsstelle, die spezifisch an ein erstes Antigen bindet, und eine zweite Bindungsstelle, die spezifisch an ein zweites Antigen bindet, umfasst, an das erste und das zweite Antigen, wobei das Verfahren die folgenden Schritte umfasst:

   - Einfangen des Antikörpers auf einer festen Phase unter Verwendung eines Einfangreagenz, das spezifisch an eine konstante Region des Antikörpers bindet,
   - Inkubieren des eingefangenen Antikörpers mit dem ersten oder zweiten Antigen in einem Puffer, umfassend 1 mM KH2PO4, 10 mM Na2HPO4, 137 mM NaCl, 2,7 mM KCl, pH 7,4, 0,05 % Tween-20 und 300 mM NaCl, um einen immobilisierten Antikörper-Antigen-Komplex zu bilden,
   - Inkubieren des Komplexes aus eingefangenem Antikörper und Antigen mit dem Antigen, das nicht zur Bildung des Komplexes aus eingefangenem Antikörper und Antigen verwendet wurde, in einem Puffer, umfassend 1 mM KH2PO4, 10 mM Na2HPO4, 137 mM NaCl, 2,7 mM KCl, pH 7,4, 0,05 % Tween-20 und 300 mM NaCl, um einen Komplexes aus eingefangenem Antikörper, Antigen und Antigen zu bilden, und
   - Bestimmen der Einzel- und darauf basierend auch der Gesamtbindung des Antikörpers an das erste und das zweite Antigen, wenn die Bildung des Komplexes aus eingefangenem Antikörper und Antigen zu einem ersten Bindungssignal führt und die Bildung des Komplexes aus eingefangenem Antikörper, Antigen und Antigen zu einem zweiten Bindungssignal führt, das gegenüber dem ersten Bindungssignal erhöht ist,
   wobei die feste Phase ein Oberflächenplasmonenresonanzchip ist, das erste Bindungssignal eine Oberflächenplasmonenresonanzantwort ist und das zweite Bindungssignal eine Oberflächenplasmonenresonanzantwort ist.

**4.** Verfahren nach einem der Ansprüche 1 bis 3, wobei der Antikörper ein bispezifischer Antikörper ist.

**5.** Verfahren nach Anspruch 4, wobei der bispezifische Antikörper ein CrossMab oder ein DutaFab ist.

**6.** Verfahren nach einem der Ansprüche 1 bis 5, wobei das Einfangreagenz ein Anti-Fc-Region-Antikörper oder ein Anti-Fab-Antikörper ist.

**7.** Verfahren nach Anspruch 6, wobei der Anti-Fab-Antikörper ein Anti-Kappa-Leichtketten-Antikörper oder ein Anti-Lambda-Leichtketten-Antikörper ist.

**8.** Verfahren nach einem der Ansprüche 1 bis 7, wobei der Antikörper ein DutaFab ist, die feste Phase ein Oberflächenplasmonenresonanzchip ist, das Einfangreagenz ein Anti-Kappa-Leichtketten-Antikörper oder ein Anti-Lambda-Leichtketten-Antikörper ist, das erste Bindungssignal eine Oberflächenplasmonenresonanzantwort ist und das zweite Bindungssignal eine Oberflächenplasmonenresonanzantwort ist.

**9.** Verfahren nach einem der Ansprüche 1 bis 8, wobei das Einfangen durch Injizieren des Antikörpers für 45 bis 720 Sekunden bei einer Flussrate von 2,5 bis 10 μl/min und bei einer Konzentration von 0,6 μg/ml bis 10 μg/ml erfolgt.

**10.** Verfahren nach Anspruch 9, wobei das Einfangen durch Injizieren des Antikörpers für etwa 60 Sekunden, bei einer Flussrate von etwa 5 μl/min und bei einer Konzentration von 0,6 μg/ml bis 10 μg/ml erfolgt.

**11.** Verfahren nach einem der Ansprüche 1 bis 10, wobei das Inkubieren durch eine Injektion des jeweiligen Antigens in einer Konzentration von 0,5 bis 5 μg/ml für 20 bis 90 Sekunden erfolgt.

**12.** Verfahren nach Anspruch 11, wobei das Inkubieren durch eine Injektion des jeweiligen Antigens in einer Konzentration von etwa 2 μg/ml für 30 oder 60 Sekunden erfolgt.

**Revendications**

**1.** Méthode de détermination de la liaison d'un anticorps, qui comprend un premier site de liaison se liant spécifiquement à un premier antigène et un second site de liaison se liant spécifiquement à un second antigène, audit premier et audit second antigène, dans laquelle la méthode comprend les étapes suivantes :

a) capture de l'anticorps sur une phase solide en utilisant un réactif de capture se liant spécifiquement à un domaine constant de l'anticorps,
b) incubation de l'anticorps capturé avec le premier ou le second antigène dans un tampon comprenant 1 mM de KH2PO4, 10 mM de Na2HPO4, 137 mM de NaCl, 2,7 mM de KCl, pH 7,4, 0,05 % de Tween-20 et 300 mM de NaCl pour former un complexe anticorps capturé-antigène et détermination d'un premier signal de liaison,
c) soit

incubation du complexe anticorps capturé-antigène avec l'antigène non utilisé pour la formation du complexe anticorps capturé-antigène pour former un complexe anticorps capturé-antigène-antigène et détermination d'un deuxième signal de liaison,
soit
régénération de la surface, capture de l'anticorps sur une phase solide en utilisant un réactif de capture se liant spécifiquement à un domaine constant de l'anticorps, incubation de l'anticorps capturé avec l'antigène non utilisé pour la formation du complexe anticorps capturé-antigène à l'étape b) dans le même tampon qu'à l'étape b) pour former un complexe anticorps capturé-antigène et détermination d'un troisième signal de liaison,
et

d) détermination de la liaison globale ou individuelle de l'anticorps au premier et au second antigène à partir du premier signal de liaison et du deuxième ou troisième signal de liaison,
dans laquelle la phase solide est une puce à résonance de plasmons de surface, le premier signal de liaison est une réponse de résonance de plasmons de surface et le deuxième signal de liaison est une réponse de résonance de plasmons de surface.

**2.** Procédé selon la revendication 1, dans lequel l'étape d) est

d) la détermination de la liaison globale ou individuelle de l'anticorps au premier et au second antigène si i) la formation du complexe anticorps capturé-antigène résulte en un premier signal de liaison et ii) la formation du complexe anticorps capturé-antigène-antigène ou la formation du complexe anticorps capturé-antigène après la régénération résulte en un deuxième ou troisième signal de liaison qui est augmenté par rapport au premier signal de liaison.

**3.** Méthode de détermination de la liaison globale et individuelle d'un anticorps, qui comprend un premier site de liaison se liant spécifiquement à un premier antigène et un second site de liaison se liant spécifiquement à un second antigène, audit premier et audit second antigène, dans laquelle la méthode comprend les étapes suivantes :

- capture de l'anticorps sur une phase solide en utilisant un réactif de capture se liant spécifiquement à une région constante de l'anticorps,
- incubation de l'anticorps capturé avec le premier ou le second antigène dans un tampon comprenant 1 mM de KH2PO4, 10 mM de Na2HPO4, 137 mM de NaCl, 2,7 mM de KCl, pH 7,4, 0,05 % de Tween-20 et 300 mM de NaCl pour former un complexe anticorps immobilisé-antigène,
- incubation du complexe anticorps capturé-antigène avec l'antigène non utilisé pour la formation du complexe anticorps capturé-antigène dans un tampon comprenant 1 mM de KH2PO4, 10 mM de Na2HPO4, 137 mM de NaCl, 2,7 mM de KCl, pH 7,4, 0,05 % de Tween-20 et 300 mM de NaCl pour former un complexe anticorps capturé-antigène-antigène,
et
- détermination de la liaison individuelle et également, sur cette base, de la liaison globale de l'anticorps au premier et au second antigène si la formation du complexe anticorps capturé-antigène résulte en un premier signal de liaison et la formation du complexe anticorps capturé-antigène-antigène résulte en un second signal de liaison qui est augmenté par rapport au premier signal de liaison,
dans laquelle la phase solide est une puce à résonance de plasmons de surface, le premier signal de liaison est une réponse de résonance de plasmons de surface et le second signal de liaison est une réponse de résonance de plasmons de surface.

**4.** Méthode selon l'une quelconque des revendications 1 à 3, dans laquelle l'anticorps est un anticorps bispécifique.

**5.** Méthode selon la revendication 4, dans laquelle l'anticorps bispécifique est un CrossMab ou un DutaFab.

**6.** Méthode selon l'une quelconque des revendications 1 à 5, dans laquelle le réactif de capture est un anticorps anti-région Fc ou un anticorps anti-Fab.

**7.** Méthode selon la revendication 6, dans laquelle l'anticorps anti-Fab est un anticorps anti-chaîne légère kappa ou un anticorps anti-chaîne légère lambda.

**8.** Méthode selon l'une quelconque des revendications 1 à 7, dans laquelle l'anticorps est un DutaFab, la phase solide est une puce à résonance de plasmons de surface, le réactif de capture est un anticorps anti-chaîne légère kappa ou un anticorps anti-chaîne légère lambda, le premier signal de liaison est une réponse de résonance de plasmons de surface et le second signal de liaison est une réponse de résonance de plasmons de surface.

**9.** Méthode selon l'une quelconque des revendications 1 à 8, dans laquelle la capture se fait par injection de l'anticorps pendant 45 à 720 secondes, à un débit de 2,5 à 10 $\mu$L/min, et à une concentration de 0,6 $\mu$g/mL à 10 $\mu$g/mL.

**10.** Méthode selon la revendication 9, dans laquelle la capture se fait par injection de l'anticorps pendant environ 60 secondes, à un débit d'environ 5 $\mu$L/min, et à une concentration de 0,6 $\mu$g/mL à 10 $\mu$g/mL.

**11.** Méthode selon l'une quelconque des revendications 1 à 10, dans laquelle l'incubation se fait par une injection de l'antigène correspondant à une concentration de 0,5 à 5 $\mu$g/mL pendant 20 à 90 secondes.

**12.** Méthode selon la revendication 11, dans laquelle l'incubation se fait par une injection de l'antigène correspondant à une concentration d'environ 2 $\mu$g/mL pendant 30 ou 60 secondes.

**Figure 1**

EP 3 519 820 B1

A

Ra

Rv

Capture
signal Rc

antibody-mediated capture

B

Ra

Rv

Capture
signal (Rc)

Regeneration

Time [s]

Response [RU]

RU
1400
1200
1000
800
600
400
200
0
-200

-100    0    100   200   300   400   500    s

Figure 2

EP 3 519 820 B1

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2010256338 A
- WO 2012023053 A
- WO 2015172800 A
- WO 9308829 A
- US 5731168 A
- WO 2009089004 A
- US 4676980 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- WO 2012163520 A
- WO 9627011 A
- WO 98050431 A
- EP 1870459 A
- WO 2007110205 A
- WO 2007147901 A
- WO 2010129304 A
- WO 201190754 A
- WO 2011143545 A
- WO 2012058768 A
- WO 2013157954 A
- WO 2013096291 A

### Non-patent literature cited in the description

- *J. Pharm. Biomed. Anal.,* 2016, vol. 132, 141-147
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **MILSTEIN, C. ; CUELLO, A.C.** *Nature,* 1983, vol. 305, 537-540
- **TRAUNECKER, A. et al.** *EMBO J.,* 1991, vol. 10, 3655-3659
- **BRENNAN, M. et al.** *Science,* 1985, vol. 229, 81-83
- **KOSTELNY, S.A. et al.** *J. Immunol.,* 1992, vol. 148, 1547-1553
- **HOLLIGER, P. et al.** *Proc. Natl. Acad. Sci. USA,* 1993, vol. 90, 6444-6448
- **GRUBER, M. et al.** *J. Immunol.,* 1994, vol. 152, 5368-5374
- **TUTT, A. et al.** *J. Immunol.,* 1991, vol. 147, 60-69
- **M. A. COOPER.** *Nature Reviews Drug Discovery,* 2002, vol. 1, 515-528
- **D. G. MYSZKA.** *Journal of Molecular Recognition,* 1999, vol. 12, 390-408
- **R. L. RICH ; D. G. MYSZKA.** *Journal of Molecular Recognition,* 2000, vol. 13, 388-407
- **D. G. MYSZKA ; R. L. RICH.** *Pharmaceutical science & technology today,* 2000, vol. 3, 310-317
- **R. KARLSSON ; A. FAELT.** *Journal of immunological methods,* 1997, vol. 200, 121-133
- **T. A. MORTON ; D. G. MYSZKA.** *Methods in enzymology,* 1998, vol. 295, 268-294
- **K. NAGATA ; H. HANDA.** Real-time analysis of biomolecular interactions. Springer, 2000
- **R. L. RICH ; D. G. MYSZKA.** *Current opinion in biotechnology,* 2000, vol. 11, 54-61
- **A. C. MALMBORG ; C. A. BORREBAECK.** *Journal of immunological methods,* 1995, vol. 183, 7-13
- **W. HUBER ; F. MUELLER.** *Current pharmaceutical design,* 2006, vol. 12, 3999-4021
- **C. GASSNER ; F. LIPSMEIER ; P. METZGER ; H. BECK ; A. SCHNUERIGER ; J. T. REGULA ; J. MOELLEKEN.** *Journal of pharmaceutical and biomedical analysis,* 2015, vol. 102, 144-149
- **YUANKUI LENG.** *Chem. Soc. Rev,* 2015, 44
- **TIAN-CAI LIU.** *Clin. Biochem.,* 2014, vol. 47, 439-444